# DEMANDE DE BREVET EUROPEEN

(11) **EP 4 840 306 A1**
(43) Date de publication de la demande: **18.11.2026**
(21) Numéro de dépôt: 25305676.6
(22) Date de dépôt: 13.05.2025
(51) Int. Cl.: A01K 67/362

(54) **DISPOSITIF D'ELEVAGE D'INSECTES**

(71) Demandeur: Entoinnov, 88150 Chavelot (FR)
(72) Inventeur: OUNNAS, Fayçal, 54130 Saint-Max (FR); CHARROIS, Emmanuel, 54740 Laneuveville-devant-Bayon (FR)
(74) Mandataire: Cabinet Bleger-Rhein-Poupon

(57) **Abrégé**

L'invention concerne un dispositif (100) d'élevage d'insectes comprenant :
- une cuve (101) configurée pour recevoir des insectes et leur substrat d'élevage,
- au moins un système d'injection configuré pour introduire de l'air (106a, 106b) et/ou de l'eau (104) et un capteur environnemental (108) dans la cuve,

Le dispositif est équipé d'au moins une vis conique (102) placée à l'intérieur de la cuve, la partie de la cuve située au-dessous de la vis étant la partie inférieure (116) de la cuve, la partie de la cuve située au-dessus de ladite vis étant la partie supérieure (112) de la cuve, et la partie de la cuve située entre les parties inférieure et supérieure étant la partie intermédiaire (114) de la cuve, ladite vis étant montée mobile en rotation au moyen d'un système d'entraînement (120) et agencée pour convoyer les insectes de la partie inférieure vers la partie supérieure de la cuve.

## Description

### DOMAINE DE L'INVENTION

La présente invention concerne un dispositif d'élevage d'insectes. En particulier, la présente invention concerne un dispositif et une méthode permettant de faciliter l'élevage et la croissance des insectes, par exemple comestibles.

### ÉTAT DE LA TECHNIQUE

Aujourd'hui, plus de 2000 espèces d'insectes comestibles ont été identifiées. Ces insectes comestibles constituent une source de matières premières, notamment pour l'alimentation animale ou humaine. Des insectes non-comestibles peuvent aussi être intéressant à élever.

L'élevage industriel de tels insectes connaît donc un essor afin de réguler et d'optimiser la croissance des insectes. Cependant, les systèmes d'élevage connus et conventionnels ne sont pas satisfaisants.

En effet, la plupart des systèmes connus reposent sur l'utilisation de bacs de faibles volumes dans lesquels les insectes se développent. L'intervention humaine lors de l'utilisation de tels systèmes est nécessaire pour le nettoyage, le nourrissage, l'hydratation et l'entretien des insectes. Une telle intervention humaine implique donc une diminution du rendement de l'élevage et un risque sanitaire (par exemple, la contamination par des virus et/ou des bactéries).

De plus, l'empilement vertical des bacs d'élevage implique une complexification de la logistique liée au traitement et à la gestion de ces bacs.

Finalement, afin de réduire le risque de contamination des insectes et d'éviter des phénomènes de cannibalisme entre les insectes adultes et les jeunes larves et/ou les nymphes, les insectes éclos sont déplacés plusieurs fois ce qui augmente d'autant plus la logistique.

Pour tenter de pallier, au moins en partie, les inconvénients des systèmes industriels conventionnels d'élevage d'insectes, la demanderesse a précédemment déposé une demande internationale de brevet WO 2023/217957, portant sur un dispositif d'élevage d'insectes, comprenant :
D'une part une cuve configurée pour recevoir des insectes ainsi que leur substrat d'élevage,
- D'autre part une vis sans fin placée dans une gaine, à l'intérieur de la cuve, ladite vis sans fin étant montée mobile en rotation au moyen d'un système d'entraînement et agencée pour convoyer les insectes depuis la partie inférieure de la cuve, vers la partie supérieure de la cuve,
- D'autre part encore, au moins un système d'injection configuré pour introduire de l'air et/ou de l'eau à l'intérieur de la cuve.
- D'autre part encore, un capteur environnemental dans la partie inférieure et/ou dans la partie intermédiaire de la cuve.

Un tel dispositif d'élevage d'insecte, sous la forme d'une cuve dans laquelle sont élevés les insectes, présente un certain nombre d'avantages, par rapport aux dispositifs sous la forme de simples bacs d'élevage qui existaient jusqu'alors.

En effet, en particulier, la mise en œuvre d'une cuve permet de fournir un volume important pour l'élevage dans un seul et unique dispositif. Cela a donc pour effet de diminuer la chaine de logistique liée à l'utilisation d'un ensemble de dispositifs de bacs présentant de faibles volumes. De plus, le rendement d'élevage est optimisé par rapport à l'espace au sol utilisé par le système.

En outre, il est également notable que l'utilisation d'un capteur environnemental permet de diminuer l'intervention humaine. En effet, l'utilisateur n'interviendra que lorsque cela sera nécessaire en fonction des données recueillies par le capteur. Enfin, l'utilisation du capteur environnemental peut aussi permettre l'automatisation de l'élevage supprimant ainsi totalement l'intervention humaine et réduisant les coûts d'utilisation.

Placer le capteur dans la partie intermédiaire de la cuve permet de mesurer les conditions environnementales à l'endroit où la population d'insectes sera la plus grande. Les données ainsi recueillies seront représentatives de l'environnement des insectes en élevage.

En ce qui concerne le système d'injection, celui-ci présente l'avantage de permettre d'hydrater et d'oxygéner le milieu d'élevage en continu, limitant ainsi l'asphyxie et la déshydratation des insectes et donc de limiter la diminution du rendement.

Cela étant, la vis sans fin agencée pour convoyer les insectes depuis le bas de la cuve vers le haut de cette dernière, bien qu'elle présente l'avantage de permettre un brassage du milieu d'élevage, évitant ainsi un gradient de densité de ce dernier entre le haut et le bas de la cuve, est responsable, par cette action de convoyage perpétuel vers le haut de l'appareil, de la mise en place d'un gradient de pression croissant depuis le haut vers le bas de la cuve.

Par conséquent, au moment où les insectes se retrouvent positionnés en partie inférieure de la cuve, ceux-ci sont susceptibles de subir une pression particulièrement importante imposée de la part du milieu d'élevage, qui est positionné à des niveaux supérieurs au sein de ladite cuve. Les conditions pour l'élevage et la croissance des insectes ne sont alors pas optimales, et il est nécessaire de trouver des solutions pour les améliorer.

En outre, il a pu être constaté que la gaine dans laquelle est emmanchée la vis sans fin est susceptible d'entraîner un engorgement en partie inférieure de la cuve, au moment de l'entrée du milieu d'élevage au sein de ladite gaine, avant que celui-ci soit convoyé depuis cette partie inférieure jusqu'à la partie supérieure de ladite cuve au moyen de l'action de la vis sans fin. Ces contraintes sont d'autant plus importantes à mesure que la densité du substrat d'élevage augmente, en sorte que les substrats qui peuvent être mis en œuvre dans une telle cuve sont limités et doivent être soigneusement sélectionnés pour éviter un tel coincement de la matière. En particulier, il est uniquement possible d'utiliser un substrat d'élevage contenant des matières fines, et présentant un taux d'humidité faible.

En outre, l'utilisation d'une forte densité d'animaux est limitée par la présence de la gaine qui entraine un cisaillement potentiellement létal envers les animaux.

La présente divulgation vient améliorer la situation, en proposant un dispositif d'élevage qui permet notamment d'optimiser les conditions de croissance des insectes au sein d'une cuve d'un dispositif d'élevage, et ainsi améliorer la productivité permise par un tel dispositif.

### RÉSUMÉ

Dans ce but, la présente invention concerne un dispositif d'élevage d'insectes comprenant
- une cuve configurée pour recevoir des insectes et leur substrat d'élevage,
- au moins une vis conique placée à l'intérieur de la cuve, la partie de la cuve située au-dessous de la vis conique étant la partie inférieure de la cuve, la partie de la cuve située au-dessus la vis conique étant la partie supérieure de la cuve, et la partie de la cuve située entre les parties inférieure et supérieure étant la partie intermédiaire de la cuve ; ladite vis conique étant montée mobile en rotation au moyen d'un système d'entraînement et agencée pour convoyer les insectes préférentiellement de la partie inférieure de la cuve vers la partie supérieure de la cuve,
- au moins un système d'injection configuré pour introduire de l'air et/ou de l'eau à l'intérieur de la cuve,
- au moins un capteur environnemental dans la partie intermédiaire de la cuve.

Tout préférentiellement, ladite vis conique s'évase progressivement sur l'ensemble de sa longueur depuis une première extrémité libre de la vis, située à proximité de la partie supérieure de la cuve, jusqu'à une deuxième extrémité de la vis, opposée à ladite première extrémité libre, et se trouvant au voisinage de la partie inférieure de ladite cuve.

Selon une autre caractéristique particulièrement avantageuse de ladite vis conique, celle-ci est constituée d'un filet et d'un arbre central de vis, ce dernier étant de dimensions constantes sur toute la longueur de ladite vis conique, tandis que la largeur du filet de la vis conique se réduit progressivement depuis la deuxième extrémité vers l'extrémité libre de ladite vis, en sorte que le diamètre de vis passe d'un premier diamètre (Ds) en partie supérieure de cuve, à un second diamètre (Di), en partie inférieure de cuve, avec Di supérieur à Ds.

La mise en œuvre, dans le dispositif d'élevage d'insectes de l'invention, d'au moins une vis conique a pour effet, en particulier, de permettre une réduction de la contrainte à laquelle les animaux sont soumis lorsqu'ils se retrouvent à la base de la cuve.

Dans un mode de réalisation avantageux, au moins la partie inférieure de la cuve est conique.

Dans un mode de réalisation avantageux, la partie inférieure de la cuve comporte en outre une trappe de vidange latérale configurée pour permettre l'évacuation des insectes et de leur substrat d'élevage.

La trappe permet de faciliter le nettoyage de la cuve et la récolte des insectes.

Dans un mode de réalisation avantageux, le volume occupé par la vis conique dans la cuve est compris entre 0,1% à 5% par rapport au volume total de ladite cuve.

En effet, cette configuration permet d'obtenir un différentiel de mélange en fonction de la distance au centre de la vis permettant ainsi aux insectes de se déplacer entre un milieu plus mélangé et un milieu moins mélangé en fonction de leurs besoins en nutriments. Il a été observé par les inventeurs qu'un tel volume de la vis compris entre 0,1% et 5% par rapport au volume total de la cuve permet d'optimiser le cycle de déplacement des insectes et donc le rendement du système.

Dans un mode de réalisation avantageux, l'au moins un système d'injection d'air comprend des injecteurs d'air répartis sur la partie intermédiaire de la cuve.

En effet, le CO₂ étant plus dense que l'air, il a tendance à s'accumuler en pied de cuve. Une rotation simple de la vis conique permet l'évacuation du CO₂ accumulé au pied de la cuve. Une injection d'air associée à la rotation de la vis par le pied de la cuve ou par la partie intermédiaire de la cuve est encore plus efficace pour renouveler l'atmosphère de la cuve.

Dans un mode de réalisation avantageux, l'au moins un système d'injection comprend des injecteurs d'eau répartis sur la partie supérieure de la cuve, les injecteurs d'eau étant avantageusement des nébuliseurs, des pulvérisateurs, des disperseurs et/ou des asperseurs d'eau.

Dans un mode de réalisation avantageux, le capteur environnemental comprend un capteur de CO₂, un capteur d'humidité, un capteur de température et/ou un capteur d'ammoniac, avantageusement placé dans la moitié inférieure de la partie intermédiaire de la cuve.

En effet, le capteur de CO₂ mesure la quantité de CO₂ présent dans le milieu d'élevage permettant, le cas échéant, de prendre des mesures afin de réduire le risque d'asphyxie. Placer le capteur de CO₂ dans la moitié inférieure de la partie intermédiaire de la cuve permet une mesure plus efficace et représentative de la quantité de CO₂ totale produite dans la cuve.

Dans un mode de réalisation avantageux, le dispositif d'élevage d'insectes comprend en outre une unité de contrôle connectée au système d'entraînement de la vis conique et au capteur environnemental.

En effet, la présence d'une unité de contrôle permet une automatisation, au moins partielle, de l'élevage réduisant ainsi l'intervention humaine et donc le risque de contamination.

En outre, l'invention concerne aussi une méthode d'élevage d'insectes comprenant les étapes suivantes :
- Introduire des insectes et leur substrat d'élevage dans un dispositif d'élevage d'insectes tel que décrit ci-dessus,
- Déterminer régulièrement au moins un paramètre environnemental dans la partie intermédiaire et ou inférieure de la cuve,
- En fonction de l'au moins un paramètre environnemental :
   ∘ Injecter de l'air et/ou de l'eau dans la cuve, et/ou
   ∘ Entrainer en rotation la vis conique pour convoyer le substrat d'élevage de la partie inférieure de la cuve vers la partie supérieure de la cuve, et/ou
   ∘ Injecter du substrat d'élevage, et/ou
   ∘ Humidifier ou déshumidifier le substrat d'élevage.

La détermination régulière des paramètres environnementaux permet d'optimiser la croissance des insectes tout en réduisant les coûts liés à la logistique. En effet, les modifications apportées au milieu d'élevage par l'injection d'air, d'eau et/ou de substrat ou par la rotation de la vis conique ne sont effectuées que lorsque cela est nécessaire.

Dans un mode de réalisation avantageux, la rotation de la vis conique est intermittente.

### DÉFINITIONS

Dans la présente invention, les termes ci-dessous sont définis de la manière suivante :
« **Insecte** » concerne tous les stades de l'évolution entre l'œuf à l'insecte adulte, en passant par la larve et la nymphe (ou pupe).
« **Milieu d'élevage** » concerne l'ensemble des éléments présents dans la cuve. Cela correspond au milieu dans lequel sont élevés les insectes. Ce milieu comprend en particulier un substrat, les insectes, l'air et l'eau contenus dans la cuve.
« **Substrat d'élevage** » concerne un milieu comprenant des nutriments nécessaires à la croissance des insectes et dans lequel les insectes peuvent s'installer et se déplacer.

### DESCRIPTION DÉTAILLÉE

La présente invention concerne un dispositif 100 d'élevage d'insectes. Plus précisément, le dispositif permet l'élevage d'insectes dans un substrat d'élevage.

Les insectes peuvent être tout type d'insecte d'élevage. De préférence, les insectes sont des insectes comestibles tels que, mais non limité à, *Dermestes ater, Dermestes magister, Alphitobius diapennus, Oryzaephilus surinamensis, Cryptolestes ferrugineus, Hermetia Illucens, Trogoderma granarium, Locusta migratoria, Gryllodes sigillatus, Acheta domesticus* ou les vers de farines tels que, par exemple, *Tribolium castaneum, Tribolium confusum, Zophobas morios, Tenebrio molitor, Gnathocerus cornutus, Tenebroides mauritanicus* ou *Ephestia kuehniella.*

Le substrat d'élevage comprend des nutriments nécessaires à la croissance des insectes. Afin de limiter les risques de contamination, le nutriment utilisé dans le substrat est préférentiellement intégré sous forme de nourriture sèche ou semi-humide. Le substrat peut contenir des résidus de l'industrie agro-alimentaire - par exemple brassicole - des déchets de meunerie comme le son de blé ou du remoulage, des déchets de l'industrie de la restauration ou des déchets banals en vue de leur compostage ou recyclage.

En outre, le substrat d'élevage peut également contenir d'autres adjuvants optionnels destinés à limiter la croissance de microorganismes nuisibles, ou à les détruire, pour éviter une contamination de la culture par ces derniers, comme des bactériostatiques, des bactéricides, ou bien encore des fongicides.

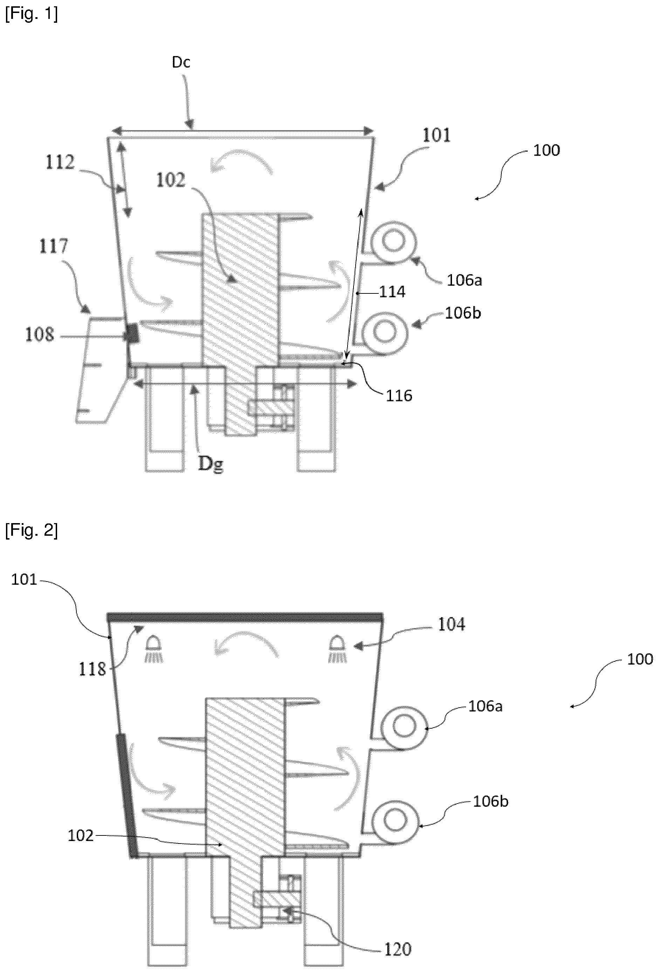

Le dispositif 100, représenté aux figures 1-3, comprend une cuve 101 configurée pour recevoir les insectes et leur substrat d'élevage, au moins une vis conique 102 placée au centre de la cuve 101, au moins un système d'injection configuré pour introduire de l'air 106a, 106b et/ou au moins un système d'injection d'eau 104 à l'intérieur de la cuve 101, et un capteur environnemental 108 dans la partie intermédiaire de la cuve 101.

A noter que, sur les figures 1 et 2 sont illustrés deux moyens d'injections d'air 106a et 106b, tandis que, sur la figure 2 sont visibles deux moyens d'injection d'eau. Toutefois, un tel mode de réalisation n'est pas limitatif du présent dispositif d'élevage d'insectes 100, et il est envisageable que ladite cuve 101 soit équipé d'un seul moyen d'injection d'air et d'un seul moyen d'injection d'eau, ou d'un nombre supérieur à deux de l'un et/ou de l'autre de ces moyens d'injection.

La cuve 101 peut être par exemple un cylindre, un parallélépipède ou un cône. Dans les modes de réalisations représentés aux figures 1 à 3, la cuve 101 possède une partie conique surmontée d'un couvercle 118, une trappe de vidange latérale 117 et est fermée à sa base.

La cuve 101 est de préférence de grande dimension afin de pouvoir contenir un grand volume d'insectes et de substrat.

Par exemple, sans toutefois que cela soit limitatif, la hauteur de la cuve 101 est comprise entre 100 centimètres et 250 centimètres, de préférence entre 150 et 200 centimètres.

Par exemple encore, la dimension principale D_{c} de la partie supérieure de la cuve est compris entre 100 centimètres et 250 centimètres, de préférence entre 100 centimètres et 200 centimètres.

La dimension principale de la partie inférieure de la cuve D_{g} est, par exemple encore, compris entre 100 centimètres et 200 centimètres.

Dans le mode de réalisation dans lequel la cuve 101 possède un contour cylindrique et une partie basale plane, le rapport entre la hauteur du contour cylindrique et le diamètre de la base peut varier. Par exemple, la hauteur du contour cylindrique peut être grande comparée au diamètre de la section de la base de la cuve comme cela est représentée aux figures 1 et 2. Alternativement, la hauteur du cylindre peut être inférieure comparée à la section de la base du cylindre hauteur de la cuve comme cela est représenté à la figure 3.

En outre, la présence d'un couvercle 118 fermant hermétiquement la cuve 101 est avantageuse. En effet, cela permet d'obtenir un milieu d'élevage contrôlé et peu sensible aux paramètres extérieurs. De plus, cela permet d'éviter l'attaque des insectes par des prédateurs. Dans le mode de réalisation dans lequel la cuve 101 possède un couvercle, une ouverture (non représentée) peut être présente dans le couvercle afin de permettre l'introduction des insectes et du substrat d'élevage dans la cuve 101.

Alternativement, la cuve 101 peut être exempte de couvercle hermétique afin de permettre l'évacuation du CO₂. Dans ce mode de réalisation, un ou plusieurs filtres peuvent être placés sur les ouvertures de la cuve 101 afin de réduire la propagation des poussières vers l'extérieur de la cuve.

Le dispositif 100 comprend aussi une vis conique 102 placée à l'intérieur de la cuve 101.

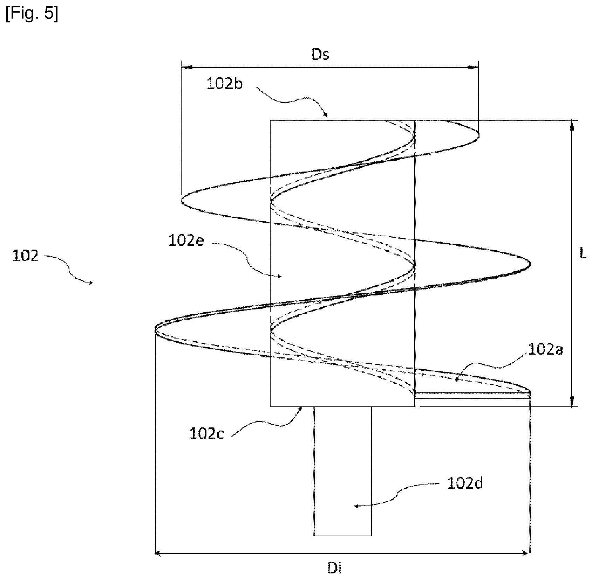

Une telle vis conique 102 est représentée, plus en détails, sur la figure 5 des dessins ci joints.

La présence d'une vis conique 102 est avantageuse notamment car elle permet un mélange efficace et homogène du milieu d'élevage. La vis conique 102 peut être fabriquée en matériau rigide ou semi-rigide.

La vis conique 102 peut être perforée, plus particulièrement au travers des pales de la vis, c'est-à-dire au niveau d'un filet 102a que comporte la vis 102, et qui sera décrit de manière plus précise ultérieurement dans la description. La perforation régulière des pales de la vis 102 est avantageuse car elle permet d'assurer une meilleure aération et un mélange du milieu d'élevage plus optimisé.

La longueur L de la vis 102 est préférentiellement inférieure à la hauteur totale de la cuve 101.

Ainsi, comme montré à la figure 2, la hauteur (correspondant à sa longueur L) et le positionnement de la vis 102 dans la cuve 101 définissent deux parties de la cuve 101 :
- une partie supérieure 112 située au-dessus de la vis 102 ;
- une partie inférieure 116 située en-dessous de la vis 102, à proximité immédiate de la paroi inférieure de la cuve 101 ; et
- et une partie intermédiaire 114 située entre la partie inférieure 116 et supérieure 112.

La figure 2 représente la cuve 101 dans une configuration verticale, et les termes « au-dessus » et « en-dessous » s'entendent en référence à l'axe vertical, qui est confondu avec l'axe de convoyage de la vis conique 102. Lorsque la cuve 101 est dans une configuration horizontale, les termes « au-dessus » et « en-dessous » s'entendent en référence au sens de convoyage de la vis conique 102 : la vis conique 102 entraine le milieu dans la partie inférieure 116 et le décharge dans la partie supérieure 112.

La vis conique 102 peut être circulaire mais elle est, encore plus préférentiellement, ellipsoïdale.

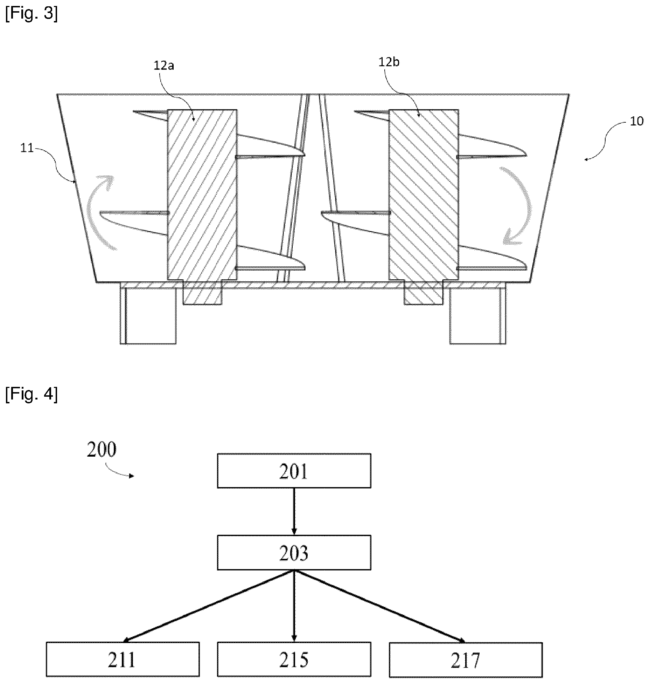

Le diamètre de la vis Di, représenté sur la figure 3, au niveau de la partie inférieure 116 de la cuve 101 (en d'autres termes au niveau du bas de la cuve 101 dans sa configuration verticale) est de préférence légèrement inférieur au diamètre Dg de la partie inférieure de la cuve 101, afin de ne pas entraver le mouvement de la vis conique 102 tout en maintenant ladite vis conique 102 en position verticale.

De manière particulièrement avantageuse, dans le dispositif d'élevage d'insectes 100 de l'invention, la conicité de ladite vis 102 est réalisée par un évasement de ladite vis 102, de manière progressive, sur l'ensemble de sa longueur L, depuis son extrémité libre 102b, à proximité de la partie supérieure 112 de la cuve 101, jusqu'à son extrémité 102c opposée à ladite extrémité libre 102b, et se trouvant au voisinage de la partie inférieure 116.

Selon un mode de réalisation particulièrement avantageux, et qui correspond à celui qui est illustré sur la figure 5 annexée, la vis conique 102 s'évase par un élargissement progressif de la largeur du filet 102a de vis, en sorte que le diamètre de vis 102 passe d'un premier diamètre Ds en partie supérieure 112 de cuve 101, à un second diamètre Di, en partie inférieure de cuve 101, avec Di supérieur à Ds (voir figure 3).

En d'autres termes, la largeur du filet 102a de vis 102 se réduit progressivement au fur et à mesure que l'on se rapproche de l'extrémité libre 102b de ladite vis 102c, opposée à l'extrémité 102c de vis au niveau de laquelle se trouve l'arbre d'entraînement 102c, et donc au fur et à mesure que l'on se rapproche de la partie supérieure 112 de la cuve 101. A noter que, parallèlement, les dimensions, notamment le diamètre, de l'arbre central 102e de vis 102 restent constantes sur toute la longueur L de ladite vis 102.

Un tel mode de réalisation, bien que particulièrement avantageux, ne doit toutefois pas être considéré comme étant limitatif de la présente invention, et la conicité de la vis 102 au sein de la cuve 101 peut également être permise par une augmentation progressive du diamètre de l'arbre central 102e de vis, depuis l'extrémité libre 102b de ladite vis 102 vers son extrémité opposée 102c, (soit toujours du haut vers le bas) associée avec un filet 102a de largeur constante sur l'ensemble de la longueur L, ou bien également avec un filet 102a de largeur croissante depuis le haut vers le bas de la cuve 101.

De préférence encore, la hauteur de la vis 102 (correspondant à sa longueur L) est inférieure à celle la cuve 101 permettant ainsi à ladite vis conique de déplacer le milieu de la base vers la partie supérieure de la cuve 101 par un mouvement hélicoïdal comme cela est montré sur les figures 1 à 3.

La vis conique 102 est montée mobile en rotation au moyen d'un système d'entraînement 120. Le système d'entrainement 120 est agencé pour mettre la vis 102 en rotation au travers de l'arbre d'entraînement 102d et ainsi convoyer les insectes de la partie inférieure 116 de la cuve vers la partie supérieure 112 de la cuve. Ainsi, lorsque la vis conique 102 est en rotation, elle emporte le mélange de substrat et d'insectes contenu dans la partie inférieure 116 pour le convoyer vers la partie supérieure 112 de la cuve par la rotation de la vis conique 102. Le déplacement du substrat et des insectes grâce à la rotation de la vis conique 102 est représenté par les flèches pleines des figures 1 à 3.

Avantageusement, la rotation de la vis conique 102 permet ainsi un mélange (ou brassage) du substrat et des insectes afin que ces derniers soient en contact permanant avec les nutriments permettant donc une diminution du cannibalisme. En outre, le brassage par la vis conique 102 permet d'éviter un gradient de densité du substrat entre la partie intermédiaire 114 et la partie inférieure 116 de la cuve. Aussi, cela évite l'accumulation des déchets tels que l'ammoniac, les déjections et le CO₂ pouvant nuire aux insectes voire les tuer.

La hauteur L de la vis conique 102 est préférentiellement légèrement inférieure à la hauteur de la cuve 101. La vis conique 102 est de préférence montée proche du centre et substantiellement parallèle à l'axe de symétrie de la cuve 101. Cela permet de créer un différentiel de mélange en fonction de la distance au centre de la vis conique 102 : les parties centrales de la cuve étant plus mélangées que les parties périphériques de la cuve permettant ainsi un renouvellement du substrat. Avantageusement, les insectes peuvent ainsi se déplacer dans le substrat afin d'atteindre des endroits plus ou moins fournis en nutriments en fonctions de leurs besoins énergétiques et donc de leur stade de croissance. Le dispositif 100 selon l'invention permet donc d'optimiser la croissance des insectes en assouvissant leurs besoins tout au long de leur croissance.

Les effets et avantages de la mise en œuvre d'une vis conique 102, conforme à ce qui a été décrit ci-dessus seront plus précisément illustrés ci-après dans l'exemple comparatif qui a été conduit entre une mise en œuvre d'un dispositif d'élevage d'insectes 100 conforme à la présente invention, et une mise en œuvre d'un dispositif d'élevage d'insectes connu dans l'état de la technique, avec une vis sans fin positionnée dans une gaine, pour le mélange du milieu d'élevage à l'intérieur de la cuve.

Le dispositif 100 comprend aussi au moins un système d'injection 106a, 106b. Dans le mode de réalisation représenté aux figures 1 à 3, le dispositif possède des systèmes d'injection 106a, 106b disposés sur des niveaux différents de la cuve 101. Dans un autre mode de réalisation non représenté, le dispositif 100 possède des systèmes d'injection positionnés au même niveau de la cuve 101.

Le système d'injection est configuré pour introduire de l'air 106a, 106b, et/ou de l'eau 104 à l'intérieur de la cuve 101. Dans le mode de réalisation dans lequel le système d'injection permet l'injection d'air (chaud ou froid, sec ou humide) dans la cuve 101, ledit système 106a, 106b, 104 est préférentiellement relié à la cuve 101 dans sa partie inférieure 116.

Dans le mode de réalisation dans lequel le système d'injection 104 permet l'injection d'eau dans la cuve, ledit système 104 est préférentiellement relié à la cuve 101 dans sa partie supérieure de la cuve 112 et le système d'injection 104 est préférentiellement un nébuliseur, un pulvérisateur, un disperseur et/ou un asperseur d'eau.

Le système d'injection 104 peut aussi être configuré pour introduire simultanément de l'air et de l'eau à l'intérieur de la cuve 101. L'introduction d'air et/ou d'eau à l'intérieur de la cuve 101 est avantageuse car elle permet d'éviter l'asphyxie et/ou la déshydratation des insectes.

Le dispositif 101 comprend en outre un capteur environnemental 108 préférentiellement, mais non limitativement, dans la partie intermédiaire 114 de la cuve 101. Le capteur environnemental 108 peut être n'importe quel type de capteur permettant de mesurer des données représentatives d'un paramètre environnemental à l'intérieur de la cuve 101. De préférence, les paramètres environnementaux mesurés ont une influence sur la reproduction, la croissance, la mortalité et les anomalies des insectes et/ou permettent de déterminer les besoins éventuels des insectes. Par exemple, les données mesurées par le capteur environnemental 108 peuvent, de manière non-limitative, être représentatives de la température ou de la densité du substrat, de la quantité de dioxyde de carbone (CO₂), de la quantité d'oxygène (O₂), de la quantité d'ammoniac (NH₃), de la quantité de composés organiques volatiles (COV) ou du taux d'humidité présents dans le substrat, de la densité d'insecte, de la quantité et/ou qualité de l'éclairement...

Le capteur environnemental 108 peut permettre de mesurer une pluralité de paramètres environnementaux.

Dans le mode de réalisation dans lequel le capteur environnemental 108 permet de mesurer des données représentatives de la quantité de CO₂ ou d'ammoniac, de l'humidité présente dans le substrat ou de la température du substrat, ledit capteur est avantageusement placé dans la moitié supérieure de la partie intermédiaire 114 de la cuve. En effet, il a été observé que c'est dans cette partie de la zone d'élevage des insectes que CO₂ et l'ammoniac sont le plus produits et où l'humidité et la température varient le plus, du fait de l'activité plus importante des insectes dans cette zone.

Avantageusement, la mesure des données représentatives de la densité d'insectes permet de contrôler la croissance, la prise de masse et/ou la mortalité des insectes. Par exemple, le capteur permettant la mesure des données représentatives de la densité d'insectes est un capteur par imagerie.

Le dispositif 100 peut comprendre en outre une unité de contrôle connectée au système d'entraînement 120 de la vis conique 102 et au capteur environnemental 108. Par exemple, l'unité de contrôle est configurée pour contrôler, par exemple à distance, la rotation de la vis conique 102 ou pour recueillir et/ou analyser les données enregistrées par le capteur environnemental 108. La connexion entre l'unité de contrôle, le système d'entraînement 120 et le capteur environnemental 108 peut être une connexion filaire ou sans fil.

La présente invention concerne aussi une méthode 200 d'élevage d'insectes. La méthode 200, représentée à la figure 4, comprend une première étape 201 d'introduction des insectes et de leur substrat d'élevage dans le dispositif 100. De préférence, une seule espèce d'insectes est introduite dans une même cuve. L'introduction est effectuée préférentiellement par le dessus de la cuve 101. Lorsque la cuve 101 est équipée d'un couvercle, l'introduction peut se faire, le cas échéant, par l'ouverture présente dans ledit couvercle.

La quantité d'insectes et de substrat introduits dépend du volume interne de la cuve 101. De préférence, la cuve 101 est remplie de substrat et d'insectes jusqu'à une hauteur ne dépassant pas la partie supérieure 112 de la cuve. Cela facilite le déversement du substrat et des insectes qui ont été convoyés par la vis conique 102.

Afin d'optimiser l'élevage des insectes (c'est-à-dire d'obtenir la plus grande quantité d'insectes dont la croissance est terminée tout en minimisant les pertes dues au cannibalisme, aux maladies...), la densité d'insectes dans le substrat est préférentiellement comprise entre 1 et 10 insectes par centimètre cube. En effet, il a été observé qu'une densité d'insectes trop faible ralentissait la croissance des insectes. A l'opposé, une densité d'insectes trop grande conduit à une agglomération des insectes impliquant aussi un ralentissement de leur croissance. Avantageusement, le grand volume de la cuve permet donc de contenir une grande quantité d'insectes augmentant ainsi le rendement tout en minimisant l'espace au sol utilisé par le dispositif.

La méthode 200 comprend en outre une deuxième étape 203 de détermination régulière d'au moins un paramètre environnemental. Cette étape 203 peut être effectuée à l'aide du capteur environnemental 108. Par exemple, l'au moins un paramètre environnemental peut-être mesuré en continu ou de manière discontinue avec une fréquence de mesure comprise entre 1 et 5 fois par jour.

En fonction des paramètres environnementaux mesurés à la deuxième étape 203, la méthode 200 comprend en outre au moins une des étapes suivantes :
- une étape 211 d'injection d'air et/ou d'eau dans la cuve 201, et/ou
- une étape 215 d'entraînement en rotation la vis conique 102 pour convoyer le substrat de la partie inférieure 116 de la cuve vers la partie supérieure 114 de la cuve, et/ou
- une étape 217 d'injection du substrat d'élevage.

Ces trois dernières étapes peuvent être déclenchée manuellement par un opérateur ou automatiquement à l'aide de l'unité de contrôle du dispositif 100.

Il est souhaitable que le taux d'humidité reste constant tout au long de la croissance des insectes. Le taux d'humidité permettant une croissance optimale dépend du type d'insecte élevé dans la cuve. Par exemple, l'hydratation des larves de *Hermetia Illucens* et *Tenebrio Molitor* n'est pas similaire. Ainsi, pour les *Hermetia Illucens,* l'étape 211 d'injection d'eau est effectuée par exemple lorsque le taux d'humidité est inférieur à 55% afin d'augmenter le taux d'humidité jusqu'à ce qu'il soit compris entre 60% et 75%. Pour les insectes de type vers de farine, l'étape 211 d'injection d'eau est effectuée par exemple lorsque le taux d'humidité, qui est un paramètre critique pour leur croissance, est inférieur à 50% afin d'augmenter le taux d'humidité jusqu'à ce qu'il soit compris entre 55% et 65%.

Dans un mode de réalisation, l'eau injectée à l'étape 211 possède une température correspondant à la température dans la cuve 101. L'eau est par exemple injectée à l'aide du système d'injection 104 dans sa partie inférieure 116 de la cuve avec un débit compris entre 1 L et 20 L par heure. La quantité d'eau introduite dépend du volume de la cuve 101. L'eau peut être introduite par nébulisation. L'injection d'eau est avantageuse car elle permet aussi d'introduire des nutriments dans le substrat.

Dans le mode de réalisation dans lequel le paramètre environnemental mesuré est le taux de CO₂, si le taux de CO₂ est supérieur à 10%, l'étape 211 d'injection d'air peut être effectuée afin de diminuer le taux de CO₂ jusqu'à ce qu'il soit compris entre 0.5% et 10%. De la même manière, les concentrations en O₂ et ammoniac peuvent être contrôlées pour maintenir de bonnes conditions de croissance des insectes.

Dans un mode de réalisation, l'air injecté à l'étape 211 possède une température correspondant à la température dans la cuve 101. L'air est par exemple injecté à l'aide du système d'injection 106a, 106b dans la partie supérieure 112 et/ou inférieure 116 de la cuve avec un débit compris entre 10 mètres cube par heure et 500 mètres cube par heure. La quantité d'air introduit dépend du volume de la cuve 101. Avantageusement, l'air injecté peut être humide permettant ainsi de modifier simultanément le taux de CO₂, d'O₂, d'ammoniac et le taux d'humidité dans la cuve.

Il est aussi souhaitable que la température du substrat reste constante tout au long de la croissance des insectes. Par exemple, lorsque la température est inférieure à +15°C, l'étape de réchauffement est effectuée. De la même manière, lorsque la température est supérieure à +35°C, l'étape de refroidissement est effectuée. La température permettant une croissance optimale dépend du type d'insecte élevé dans la cuve. Par exemple, pour les *Hermetia Illucens,* la méthode 200 peut optionnellement comprendre une étape de refroidissement ou de réchauffement afin que la température du substrat, qui est un paramètre critique pour leur croissance, soit comprise entre +26°C et +32°C. Pour les insectes de type vers de farine (*Tenebrio Molitor*)*,* la méthode 200 peut optionnellement comprendre une étape de refroidissement ou de réchauffement afin que la température du substrat soit comprise entre +25°C et +30°C. L'étape de refroidissement ou de réchauffement est par exemple effectuée par injection d'air froid ou chaud respectivement.

Finalement, dans un autre exemple encore, lorsque le paramètre environnemental mesuré est la quantité et/ou qualité de l'éclairement, la méthode 200 peut optionnellement comprendre une étape de variation de la quantité lumineuse émise dans la cuve. Ceci est avantageux car cela permet de contrôler la croissance et la reproduction des *Hermetia Illucens* grâce à la simulation de cycles jour/nuit. Au contraire, pour les insectes de type vers de farine, une obscurité totale est préférable.

Lorsque l'étape 217 d'injection de substrat est effectuée, le substrat d'élevage est préférentiellement injecté par le dessus de la cuve, par exemple, par l'ouverture présente dans le couvercle 118. Alternativement ou en combinaison avec le mode de réalisation précédent, le substrat est injecté dans la partie inférieure 116 de la cuve ou dans la partie intermédiaire 114 de la cuve 101. Les injections de substrats sont effectuées afin d'augmenter le volume accessibles aux larves lorsqu'elles grossissent (engraissement), de manière à en contrôler la densité. En effet, comme décrit plus haut, la croissance de certains insectes est influencée par la densité d'insectes et leurs interactions. L'injection de substrat est préférentiellement effectuée entre 1 et 6 fois par mois.

Les nutriments, sous forme liquide ou solide, peuvent être introduits dans la cuve avec le substrat durant l'étape 217. Alternativement, les nutriments peuvent être introduits indépendamment du substrat. Les nutriments peuvent alors être injectés à la base de la vis conique 102 afin d'être convoyé vers la partie supérieure de la cuve par la rotation de la vis 102. Lorsque les nutriments sont sous forme liquide, les nutriments sont préférentiellement introduits par le dessus de la cuve.

Avantageusement, la mesure de la température permet en outre de déterminer le besoin en nutriment des insectes. En effet, les insectes sont des animaux poïkilothermes (à sang froid). Le dégagement de chaleur par les insectes est donc lié à leur métabolisme, au frottement entre eux et à leurs mouvements. Les variations de la température du substrat provoqués par le dégagement de chaleur des insectes sont donc étroitement liées à leur métabolisme. Ainsi, lorsque la température du substrat baisse, cela indique une diminution du métabolisme des insectes et donc un besoin de nourriture. La mesure de la température peut donc conditionner le déclenchement de l'étape 217 d'injection de substrat ou l'introduction de nutriment seul afin de maintenir une croissance optimale.

L'air, l'eau et/ou le substrat injectés aux étapes 211 et 217 peuvent être mélangés au substrat et aux insectes présents auparavant dans la cuve à l'aide de la vis conique 102 entraînée en rotation durant l'étape 215. Avantageusement, l'entraînement en rotation de la vis conique 102 permet en outre d'évacuer le CO₂ et/ou l'ammoniac présents dans la cuve et/ou de modifier la densité du substrat d'élevage. L'entraînement en rotation peut se faire à l'aide du système d'entraînement 120 du dispositif 100. L'entraînement en rotation peut être intermittent, c'est-à-dire que, durant l'étape 215, la vis conique 102 peut être mise en rotation puis immobilisée et ce de manière successive. La durée et/ou la fréquence de rotation dépend du volume de la cuve 101. Par exemple, la durée totale de rotation durant l'étape 215 est comprise entre 6 secondes et 60 minutes. L'entrainement en rotation de la vis peut être continu, c'est-à-dire, durant l'étape 215, la vis conique 102 peut être mise en rotation continue.

Les insectes introduits à l'étape 201 dans la cuve 101 y séjournent pendant une période prédéterminée afin d'atteindre la croissance souhaitée. Par exemple, lorsque les insectes sont des vers de farine (*Tenebrio Molitor*)*,* la période prédéterminée est préférentiellement de 1 à 2 mois afin d'atteindre la taille maximale des larves avant la nymphose. Pour les larves d*'Hermetia Illucens,* la période prédéterminée est préférentiellement de 7 à 20 jours afin d'atteindre la taille maximale des larves avant la nymphose. La deuxième étape 203 de détermination régulière de paramètre(s) environnemental(aux) est effectuée tout au long de cette période prédéterminée. Les étapes d'injection 211 d'air et/ou d'eau, d'entraînement 215 en rotation la vis conique 102 et/ou d'injection 217 du substrat d'élevage peuvent être effectuées de manière répétée tout au long de cette période prédéterminée.

A la fin de la période prédéterminée, les insectes et le substrat peuvent être retirés de la cuve, par exemple via la trappe 117 du dispositif 100.

### DESCRIPTION DES FIGURES

[Fig. 1] Figure 1 est un schéma représentant le dispositif d'élevage d'insectes selon un mode de réalisation de l'invention.
[Fig. 2] Figure 2 représente la division en deux parties de la cuve du dispositif d'élevage d'insectes selon un autre mode de réalisation de l'invention.
[Fig. 3] Figure 3 est un schéma représentant le dispositif d'élevage d'insectes selon un autre mode de réalisation de l'invention, dans lequel une cuve d'un dispositif d'élevage d'insectes est équipée de deux vis coniques adjacentes l'une à l'autre.
[Fig. 4] Figure 4 est un diagramme représentant la méthode d'élevage d'insectes selon un mode de réalisation de l'invention.
[Fig. 5] Figure 5 illustre, de manière schématique, un mode de réalisation particulier d'un élément du dispositif d'élevage d'insectes de l'invention, à savoir un mode de réalisation particulier de la vis conique dont est équipée la cuve dudit dispositif.

### EXEMPLE

On a réalisé une campagne d'élevage des vers de farine dans une cuve 101 d'un dispositif d'élevage d'insectes 100, ladite cuve 101 comprenant une partie plane au fond surmontée d'une partie cylindrique, et étant équipée d'une vis conique 102. Les dimensions de la cuve sont 160 cm de haut et 200 cm de diamètre de la partie supérieure de la cuve. La cuve possède aussi des capteurs environnementaux placés à une hauteur de 20 cm du fond de la cuve 101 ainsi qu'un injecteur d'eau placé dans la partie supérieure à une distance d'au moins 50 cm du centre de la cuve et deux injecteurs d'air placé dans la partie inférieure de la cuve à une hauteur de 20 cm et 40 cm.

5000 g de mélange d'œufs et de larves de vers de farine de taille inférieure à 1 cm sont introduits dans une cuve 101 contenant 1000 kg de substrat constitué de drêches de brasserie.

Dans cet exemple, les nutriments utilisés sont des déchets de l'industrie brassicole. Ils sont les constituants du substrat et introduits dans la cuve 101 tout au long de leur croissance. Ainsi, au cours de la croissance des insectes, environ 200 kg de substrat seront introduits dans la cuve 101 afin d'apporter les nutriments nécessaires.

La température, le taux d'humidité, le taux de CO₂ et la quantité d'ammoniac contenus dans la cuve 101 sont mesurés au minimum 1 fois par jour à l'aide du capteur environnemental 108.

Les étapes 211 d'injection d'air et/ou d'eau dans la cuve 101, 215 d'entraînement en rotation la vis conique 102 pour malaxer et homogénéiser le milieu d'élevage, et 217 d'injection du substrat d'élevage sont effectués en fonction des paramètres mesurés.

Lorsque le taux d'humidité est inférieur à 55%, l'étape 211 d'injection d'eau dans la cuve 101 est effectuée afin d'augmenter le taux d'humidité jusqu'à ce qu'il soit compris entre 55% et 65%.

Lorsque la température est inférieure à +18°C, l'étape de réchauffement est effectuée par injection d'air chaud et lorsque la température est supérieure à +35°C, l'étape de refroidissement est effectuée par injection d'air froid afin que la température du substrat soit comprise entre +25°C et +30°C.

Lorsque le taux de CO₂ est supérieur à 10%, l'étape 211 d'injection d'air est effectuée afin de diminuer le taux de CO₂ jusqu'à ce qu'il soit compris entre 0.5% et 10%.

Après une période prédéterminée de 2 mois, les insectes sont matures et mesurent entre 2,5 cm et 3,5 cm. La cuve 101 contient ainsi 500 kg d'insectes prêts à récolter.

Le taux de mortalité est évalué entre 0 et 20%.

A titre de comparaison, une campagne d'élevage des vers de farine dans une cuve comprenant une partie plane au fond surmontée d'une partie cylindrique, et dans laquelle est positionnée une vis sans fin dans une gaine pour un brassage du milieu d'élevage, a également été effectuée, dans des conditions de croissance similaires.

Plus précisément, dans cet exemple comparatif, 5000 g de mélange d'œufs et de larves de vers de farine de taille inférieure à 1 cm sont introduits dans une cuve avec vis sans fin contenant 100 kg de substrat constitué de drêches de brasserie.

Dans cet exemple, les nutriments utilisés sont des déchets de l'industrie brassicole. Ils sont les constituants du substrat et introduits dans la cuve avec vis sans fin tout au long de leur croissance. Il a pu être déterminé que, au cours de la croissance des insectes, environ 400 kg de substrat doivent être introduits dans la cuve avec vis sans fin, afin d'apporter les nutriments nécessaires.

La température, le taux d'humidité, le taux de CO₂ et la quantité d'ammoniac contenus dans la cuve avec vis sans fin sont mesurés au minimum 1 fois par jour à l'aide d'un capteur environnemental. Les étapes d'injection d'air et/ou d'eau dans la cuve avec vis sans fin, d'entraînement en rotation la vis sans fin et d'injection du substrat d'élevage sont effectués en fonction des paramètres mesurés.

Lorsque le taux d'humidité est inférieur à 50%, l'étape d'injection d'eau dans la cuve avec vis sans fin est effectuée afin d'augmenter le taux d'humidité jusqu'à ce qu'il soit compris entre 55% et 65%.

Lorsque la température est inférieure à +18°C, l'étape de réchauffement est effectuée par injection d'air chaud et lorsque la température est supérieure à +35°C, l'étape de refroidissement est effectuée par injection d'air froid afin que la température du substrat soit comprise entre +25°C et +30°C.

Lorsque le taux de CO₂ est supérieur à 10%, l'étape d'injection d'air est effectuée afin de diminuer le taux de CO₂ jusqu'à ce qu'il soit compris entre 0.5% et 10%.

Après une période prédéterminée de 2 mois, les insectes sont matures et mesurent entre 2,5 cm et 3,5 cm. Il a pu alors être déterminé que, dans le dispositif d'élevage muni d'une cuve avec vis sans fin, une quantité de 50 kg d'insectes prêts à récolter a été obtenue.

Il ressort de ce qui précède que, au moyen du dispositif pour l'élevage d'insectes selon la présente invention, et dont la cuve est équipée d'une vis conique, sans que celle-ci soit emmanchée dans une gaine, la production d'insectes à maturité est multipliée par 10 (500 kg d'insectes prêts à être récoltés) en comparaison avec le dispositif d'élevage d'insectes précédemment connu, dans lequel une vis sans fin est placée dans une gaine à l'intérieur de la cuve (50 kg d'insectes prêts à être récoltés), pour :
- Des conditions d'élevage similaires en termes de taux d'humidité, de température et de taux de CO₂,
- Un substrat de départ identiques (constitué de drêches de brasserie),
- une quantité d'œufs et de larves ensemencés en début de process dix fois moindre (5000 g de mélange d'œufs et de larves de vers de farine de taille inférieure à 1 cm),
- Avec, par ailleurs, une quantité de substrat introduite tout au long du process deux fois moindre (200 kg de substrat ont dû être introduits dans le présent dispositif d'élevage dans lequel une vis conique est positionnée à l'intérieur de la cuve en comparaison avec une quantité supérieure de 400 kg qui a dû être introduite dans le dispositif d'élevage connu où la vis est sans fin et dans une gaine, du fait que la capacité de stockage initiale dans la cuve présentant une vis conique est plus importante).

La vis conique 102 dans le dispositif d'élevage d'insectes 100 de l'invention permet en effet un meilleur support de la matière sur la totalité de la hauteur d l'élevage. Par conséquent, la pression appliquée par le milieu d'élevage est réduite à travers la forme particulière conique de la vis, et les animaux qui se trouvent à la base du bioréacteur 100 sont moins contraints par le poids de cette matière, en comparaison avec un dispositif équipée d'une vis sans fin dans une gaine.

En outre, la mise en œuvre, dans un dispositif d'élevage d'insectes 100 de l'invention, d'une vis conique 102, permet l'utilisation d'un spectre de substrat d'élevage plus important, avec une granulométrie large, et une humidité plus importante, alors que la vis sans fin dans une gaine ne permettait que l'utilisation de substrats sous la forme de matières fines, avec une humidité faible.

Également, la vis conique 102 permet une homogénéisation des animaux et des matières (substrat d'élevage ou hydratation), plus rapide, dans l'intégralité du volume de la cuve, bien que celle-ci puisse être de capacité supérieure en comparaison avec la cuve équipée d'une vis sans fin dans une gaine, dans laquelle le milieu est aspiré à l'intérieur de la gaine en partie inférieure de la cuve pour être ramené vers la partie supérieure, aboutissant à un temps nécessaire à l'homogénéisation supérieur.

Finalement, le dispositif d'élevage d'insectes muni d'une vis conique permet, de manière particulièrement avantageuse, des volumes d'élevages supérieurs. En effet, plusieurs bioréacteurs peuvent être fusionnés afin d'augmenter les volumes d'élevage, que ce soit de manière verticale ou horizontale.

Ainsi, en référence à présent à la figure 4 annexée, la présente invention est également relative à un dispositif d'élevage d'insectes 10 comportant au moins, au sein d'une seule cuve 11 de dimensions adaptées, deux vis coniques 12a, 12b.

Un tel dispositif d'élevage d'insectes 10 peut également comporter un nombre supérieur à deux vis coniques, par exemple trois, voire quatre vis coniques en série, afin de permettre une augmentation encore plus importante du volume d'élevage.

A noter que l'ensemble des caractéristiques techniques qui ont été décrites précédemment en lien avec le dispositif d'élevage d'insectes 100 présentant une seule vis conique 102 sont applicables au dispositif d'élevage d'insectes 10 présentant deux vis coniques 12a et 12b ou plus, à savoir la présence d'un ou plusieurs capteur(s), de système(s) d'injection d'air et/ou d'eau, la trappe de vidange, le couvercle, ou tout autre élément décrit ci-dessus.

Dans un tel dispositif d'élevage d'insectes comportant une pluralité de vis coniques, ces dernières peuvent entrainer de la matière entre elles.

Une telle démultiplication du nombre de vis de mélange dans un dispositif d'élevage d'insectes n'est pas envisageable lorsque l'élément mélangeur consiste en une vis sans fin dans une gaine. En effet, cela aboutirait nécessairement encore à une augmentation de la contrainte par pression à laquelle sont soumis les animaux lorsqu'ils se trouvent dans la partie inférieure de la cuve dudit dispositif, en particulier lorsque ceux-ci se trouvent dans le volume de ladite cuve situé entre deux vis sans fin.

Enfin, un autre avantage du dispositif d'élevage d'insecte, 10 ou 100, de la présente invention, réside dans le fait qu'il permet une simplification du remplissage et de la vidange, les ouvertures d'un tel dispositif étant de dimensions plus importantes, étant donné que lesdits dispositifs 10, 100, peuvent être de dimensions plus importantes en comparaison avec les dispositifs existants.

### REFERENCES NUMERIQUES

100 - Dispositif d'élevage d'insectes
101 - Cuve
102 - Vis conique
102a - Filet de la vis conique
102b - Extrémité libre de la vis conique
102c - Extrémité de la vis conique reliée à l'arbre d'entraînement
102d - Arbre d'entraînement de la vis conique
102e - Arbre central de la vis conique
Ds - premier diamètre de la vis conique au niveau de son extrémité libre
Di - second diamètre de la vis conique au niveau de son extrémité reliée à l'arbre d'entraînement
104 -Système d'injection d'eau
106a et 106b - Système d'injection d'air
108 - Capteur environnemental
117 - Trappe
112 - Partie supérieure de la cuve
116 - Partie inférieure de la cuve
118 - Couvercle
120 - Moteur d'entrainement de la vis conique
D_{c} - Dimension inférieure de la cuve
D_{g} - Dimension supérieure de la cuve
200 - Méthode d'élevage d'insectes
201 - Introduction des insectes et leur substrat d'élevage dans le dispositif d'élevage
203 - Détermination régulière d'au moins un paramètre environnemental dans la partie intermédiaire de la cuve
211 - Injection d'air et/ou d'eau dans la cuve
215 - Entrainement en rotation de la vis conique
217 - Injection de substrat d'élevage

## Revendications

1. Dispositif (100) d'élevage d'insectes comprenant, au moins :
- une cuve (101) configurée pour recevoir des insectes et leur substrat d'élevage,
- au moins un système d'injection configuré pour introduire de l'air (106a, 106b) et/ou de l'eau (104) à l'intérieur de la cuve (101), et
- un capteur environnemental (108) dans la partie intermédiaire (114) de la cuve (101),
ledit dispositif (100) étant **caractérisé en ce qu'**il est équipé d'au moins une vis conique (102) placée à l'intérieur de la cuve (101), la partie de la cuve (101) située au-dessous de la vis conique (102) étant la partie inférieure (116) de la cuve (101), la partie de la cuve (101) située au-dessus de la vis conique (102) étant la partie supérieure (112) de la cuve(101), et la partie de la cuve (101) située entre les parties inférieure (116) et supérieure (112) étant la partie intermédiaire (114) de la cuve (101), ladite vis conique (102) étant montée mobile en rotation au moyen d'un système d'entraînement (120) et agencée pour convoyer les insectes de la partie inférieure (116) de la cuve (101) vers la partie supérieure (112) de celle-ci.

2. Dispositif (100) d'élevage d'insectes selon la revendication 1, **caractérisé en ce que** ladite vis conique (102) s'évase progressivement sur l'ensemble de sa longueur (L) depuis une première extrémité libre (102b) de la vis (102), située à proximité de la partie supérieure (112) de la cuve (101), jusqu'à une deuxième extrémité (102c) de la vis (102), opposée à ladite première extrémité libre (102b), et se trouvant au voisinage de la partie inférieure (116) de ladite cuve (101).

3. Dispositif (100) d'élevage d'insectes selon la revendication 2, **caractérisé en ce que** ladite vis conique (102) est constituée d'un filet (102a) et d'un arbre central de vis (102e), ce dernier étant de dimensions constantes sur toute la longueur (L) de ladite vis conique (102), tandis que la largeur du filet (102a) de la vis conique (102) se réduit progressivement depuis la deuxième extrémité (102c) vers l'extrémité libre (102b) de ladite vis (102), en sorte que le diamètre de vis (102) passe d'un premier diamètre (Ds) en partie supérieure (112) de cuve (101), à un second diamètre (Di), en partie inférieure de cuve (101), avec Di supérieur à Ds.

4. Dispositif (100) d'élevage d'insectes selon l'une quelconque des revendications 1 à 3, **caractérisé en ce que** au moins la partie inférieure (116) de la cuve est conique.

5. Dispositif (100) d'élevage d'insectes selon l'une quelconque des revendications 1 à 4, **caractérisé en ce que** la partie intermédiaire (114) de la cuve comporte en outre une trappe de vidange latérale (117) configurée pour permettre l'évacuation des insectes et de leur substrat d'élevage.

6. Dispositif (100) d'élevage d'insectes selon l'une quelconque des revendications 1 à 5, **caractérisé en ce que** ledit au moins un système d'injection d'air comprend des injecteurs d'air (106a, 106b) répartis sur la partie intermédiaire (114) de la cuve.

7. Dispositif (100) d'élevage d'insectes selon l'une quelconque des revendications 1 à 6, **caractérisé en ce que** ledit au moins un système d'injection d'eau (104) comporte des injecteurs d'eau localisés en partie supérieure (112) de la cuve (101), lesdits injecteurs consistant en des nébuliseurs, des pulvérisateurs, des disperseurs et/ou des asperseurs d'eau.

8. Dispositif (100) d'élevage d'insectes selon l'une quelconque des revendications 1 à 7, **caractérisé en ce que** le capteur environnemental (108) comprend un capteur de CO₂, un capteur d'humidité, un capteur de température et/ou un capteur d'ammoniac,.

9. Dispositif (100) d'élevage d'insectes selon l'une quelconque des revendications 1 à 8, **caractérisé en ce qu'**il comprend en outre une unité de contrôle connectée au système d'entraînement de la vis conique (102) et au capteur environnemental (108).

10. Méthode (200) d'élevage d'insectes comprenant les étapes suivantes :
- Introduire (201) des insectes et leur substrat d'élevage dans un dispositif (100) d'élevage d'insectes selon l'une quelconque des revendications 1 à 9,
- Déterminer (203) régulièrement au moins un paramètre environnemental dans la partie intermédiaire (114) de la cuve,
- En fonction de l'au moins un paramètre environnemental :
∘ Injecter (211) de l'air et/ou de l'eau dans la cuve (101), et/ou
∘ Entrainer (215) en rotation la vis conique (102) pour convoyer le substrat d'élevage de la partie inférieure (116) de la cuve vers la partie supérieure (112) de la cuve, et/ou
∘ Injecter (217) du substrat d'élevage.

11. Méthode (200) d'élevage d'insectes selon la revendication 10, dans laquelle la rotation de la vis conique (102) est intermittente.
